# EUROPEAN PATENT SPECIFICATION

(11) **EP 0 505 450 B1**
(45) Date of publication and mention of the grant of the patent: **08.11.1995**
(21) Application number: 91901322.7
(22) Date of filing: 17.12.1990
(51) Int. Cl.: A61B 5/00

(54) **APPARATUS AND METHOD FOR DETECTING EFFECTS INDICATING PHYSICAL STATE OF HEALTH AND FITNESS**
GERÄT UND VERFAHREN ZUR BESTIMMUNG DES GESUNDHEITSZUSTANDES UND DER PHYSIOLOGISCHEN LEISTUNGSFÄHIGKEIT
APPAREIL ET PROCEDE DE DETECTION DES EFFETS INDICATEURS DE L'ETAT DE SANTE ET DE LA FORME PHYSIQUE

(30) Priority: 18.12.1989 HU 664589
(43) Date of publication of application: 30.09.1992
(73) Proprietor: EGELY, György, H-2092 Budakeszi (HU); DUS, Magdolna, H-2092 Budakeszi (HU)
(72) Inventor: EGELY, György, H-2092 Budakeszi (HU); DUS, Magdolna, H-2092 Budakeszi (HU)
(74) Representative: Viering, Jentschura & Partner
(86) International application number: HU9000081
(87) International publication number: WO9108703

(56) References cited:
- AT-B- 0 328 760
- CH-A- 0 437 876
- DE-A- 1 523 246
- DE-C- 0 666 306
- US-A- 0 758 291

## Description

### Field of the invention

The invention relates to an apparatus and method for detecting and measuring physical phenomena arising in the direct environment of human body or parts thereof, showing a correlation with general state of health, fitness and simultaneously with concentrating ability.

### Prior art

In medical practice several methods using instruments have been known, so e.g. measuring blood-pressure, EKG and electroencephalographic tests. These types of examination reflect the function of one or more organs. For examining general physical condition, reflex ability, quickness for solving some test (e.g. pressing some button upon a flash of a lamp), or pupil reflex used to be applied, in other cases electric conductivity of the skin or vital capacity have been tested. Each of said examinations is restricted but on the observeation of a case taken at random, furtheron, correlation between the actually measured parameter and the characteristics intended to be measured could not be cleared unambiguously.

### Disclosure of the invention

The aim of the invention is to develop a simple testing method by using a cheap easily producible apparatus which delivers a direct information on general state and condition of health, fitness of people as well as their concentration ability well reflecting all the afore-mentioned features.

The invention is based on the recognition that upon the proximity of human body e.g. the hand, in the surrounding space an effect inducing whirls arises which is able to put some liquid into a whirling motion.

This phenomenon can be demonstrated by strewing some indicating substance onto the liquid. This physical effect affects solid objects too, supposed, the object is of small mass, i.e. inertia is inconsiderable, at the same time it is arranged movably and rotatably.

In course of control tests we arrived at the conclusion that said effect cannot be replaced by artificially established physical effects or fields of force (thermic effect, magnetic field, field of electrostatic forces etc.), none of the last mentioned yield the result as it might be achieved by the physical effect according to the invention.

We arrived at the recognition that the physical effect according to the invention - although the nature thereof is unknown for us - in respect to the intensity of the torque or motion having been induced is staying in connection with individual natural endowments and the state in course of testing, i.e. general conditions of health and fitness and concentrating ability.

We also arrived at the recognition in so far as torque or mechanic motion raised by the body, limbs or members of the body of a person tested can be numerically defined by reading number of revolutions and can be advantageously used for demonstrating, numerical measurement and characterizing the effect of some favourable or unfavourable (disadvantageous) circumstance changing individual natural endowments, general physical conditions or fitness of the person tested.

Accordingly, the invention relates to an apparatus for indicating and measuring the physical effect being in connection with general state of health and fitness of human beings; the most simple embodiment of the apparatus according to the invention consists of a rotor having been arranged rotatably on a frame, the mass of the rotor amounts to 0,1-2,0 g, more accurately 0,3-0,8 g, while its relative magnetic permeability is less than 50 and at least one marking is formed on the surface or flange thereof and cooperates with means for reading number of turns.

With a more complicated embodiment of the apparatus the rotor is enclosed by a stator in form of a cylinder jacket, the part of the stator - forming a circular arc of 160-200° - is a block made of metal, while the remaining part is a window made of a non-metallic material provided with an airgap.

Expediently relative dielectric coefficient of the rotor is greater than 2,5.

Rotor is made expediently of paper or some synthetic material.

Expediently the relative magnetic permeability of the rotor is less than 1.

The rotor may have the shape of a disc, cross or star, it is considered as most advantageous, if it is formed as means with lightened parts, e.g. the shape of a spoked wheel.

With a preferred embodiment of the rotor on the edge of the spoked wheel there is a mantle of the shape of a cylindrical jacket, with which generatrices are running parallel with the axis.

With another preferred embodiment of the rotor it consists of several coaxial discs.

The rotor is arranged on the axis by means of the hub-part arranged centrally on the rotor and so, in so far as the hub-part is lined with a calotte-shaped carrier made of some hard material, e.g. agate, and bears up against therethrough onto the axis.

A further possibility lies in suspending by means of a torsional spring or a filament. Strained hair or string suspension widely used in geodesical measuring apparatuses or galvanometers represents a reliable solution.

Preferably specific weight of the block amounts at least to 7.

Relative magnetic permeability of the material of the block amounts to preferably to at least 2,10⁴.

It is considered as advantageous, if the block is double-walled with a cavity in between, filled with some filler through the filling means therein, e.g. with water, oil or sugar.

With a preferred embodiment of the cavity of the block, the cavity is subdivided into cavity-parts by means of lamellae.

With a most efficient embodiment of the appartus according to the invention, circumferentially on the edge of the rotor in one or more rows below each other there are arbors formed, directed to the stator and on the inner wall of the block, in the height of the rotor said arbors are directed to the rotor, wherein arbors of the rotor are arranged between the arborrows of the block and vice versa.

With another preferred embodiment on the inner wall of the block, in the height of the rotor there is a recess formed (or a plurality of recesses lying below one another) while the edge of the rotor penetrates into said recess(es).

For measuring torque between the rotor and the frame a spring, e.g. a torsional filament is arranged. For reading the deflection of the rotor from its neutral position preferably optical means, e.g. a scale or a code-disc applied to the rotor can be used.

Preferably the apparatus can be provided with a revolution counter, too, with a preferable embodiment thereof the marking or code disk is to be found on the rotor, if the rotor is made of some transparent material and there is one (are more) spot-like or strip-shaped markings impermeable to light, while with opaque rotors one or more perforations are to be found and below the path of rotation of said perforations there is a photodiode with a pulse-counter, processor and display connected therewith.

Accordingly, our invention relates to a method for demonstrating and measuring physical effects arising in the proximity of a human body or a member of body of the person tested being in connection with the general state of health and fitness; the essential feature lies in that the above apparatus containing a non-magnetic rotor having been arranged rotatably on a frame, is set up on a place being free of draught and vibration, thereafter a member of the person to be tested, e.g. the hand is placed near to the apparatus, in a distance of 8 cm or less, kept there for at least 4 to 5 minutes, simultaneously reading constant number of revolutions of the rotor.

It becomes possible to proceed so, in so far as we repeat measuring the person tested for a longer period and by comparing results having been gained on different occasions we conclude on the fitness, fatigue or even disease.

We may proceed so, in so far as the person tested undergoes some cure (physical or mental invigorating cure), meanwhile results of measurement according to the invention indicate progress and success of the cure.

With a preferred mode of realization of the method according to the invention a plurality of persons are examined parallel and on basis opf measuring data we may conclude on persons with increased concentrating ability.

The invention will be described in detail by means of a preferred embodiment, with reference to the drawings enclosed, wherein:
- Figure 1: is the view of a preferred embodiment of the apparatus according to the invention, showing partly the window-part,
- Figure 2: is the sectional view of the suspended rotor,
- Figure 3: view of the rotor with jacket and arbors,
- Figure 4: a further possible embodiment of the rotor with several axial discs,
- Figure 5: cavity of the block and the embodiment with the cavity subdivided with lamellae,
- Figure 6: fitting of the rotor according to Figure 4 to the block,
- Figure 7: fitting of the rotor according to Figure 3 to the block,
- Figure 8: schematical view of a possible solution of the revolution-counter.

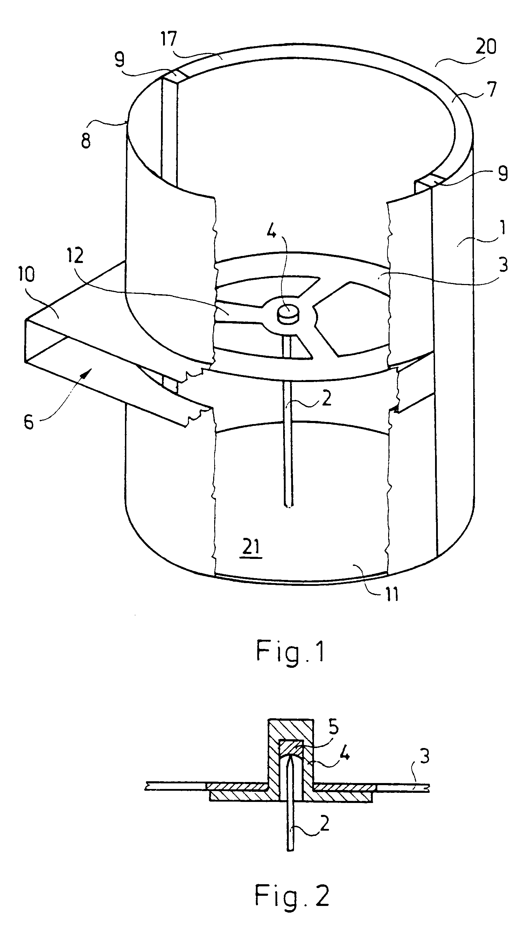

As it becomes obvious from figure 1, the apparatus 20 consists of the rotor 3 arranged on the frame 11 and the enclosing stator 1. Essentially the consists of the rotor 3 arranged on the frame 11 and the enclosing stator 1. Essentially the frame 11 is a shaft 2 arranged in the socket 21, on the top of the shaft the rotor 3 is arranged in a rotatable way. Stator 1 encloses the rotor in form of a cylinder jacket, while in half-part of the cylinder-jacket the block 7 forms the stator, the other part is the window 8. In the plane of the rotor 3, on the window there is an airgap 6, surrounded by the frame 10. In the centre of the rotor there is the hub 4, therethrough the rotor 3 is bearing up against the shaft 2.

The apparatus 20 can be made without the stator 1, in this case it is most sensitive to external influences, so draught, it can be used under special conditions only and on special places.

Block 7 of the stator 1 should be made of some metal, possibly of any material with a large specific weight, so iron or lead, preferably the block is some magnetizable material, e.g. iron, while the window is made of some synthetic material, preferably plexiglass. If the whole cylinder jacket, i.e. the block of the stator 1 is made of metal, sensitivity of the apparatus is minimal, only people with high-grade ability of exerting some effect will be able to produce some motion, frequently without success. The situation is the same, when entirety of the stator 1 is formed by the window 8. In case, if a stator is used, formation of the airgap 6 is imperative, as without the airgap the rotor 3 will deflect to any direction, but no rotary motion is taking place. Increasing dimension of the airgap 6 improves sensitivity, at the same time disturbing effects become possible. Optimal solution lies in the air-gap 6 having been cut crosswise in the window 8, lying in the height of the rotor 3 and enclosed by the frame 10.

By increasing wall-thickness of the block by 10 to 20%, sensitivity of the apparatus can be increased, however, it proves effectual only up to a wall-thickness of 8-16 mm. With double-walled hollow blocks 7 cavity 17 does not exert any direct influence on results, in this case thicknesses of the two walls of the block 7 are summarized. Cavity 17 is provided with filling means 9. If the cavity 17 is filled with some filler, e.g. water, oil or sugar, sensitivity can be increased by 10 to 20%.

Rotor may be prepared of paper or any synthetic material. The requirement lies in that it should be properly light with a low inertia, at the same time properly stiff and showing proper retentivity.

The rotor must not be magnetic or magnetizable. As it is to be seen in figure 1, the rotor 3 is a wheel connected by spokes 12 to the hub 4. From the point of view of obtainable number of revolutions synthetic material seems to be more favourable, than paper. In such a manner using PVC, by 20% better results can be achieved, than with paper, inspite of the fact that the rotor made of PVC is heavier, than that made of paper.

According to experiences size of the rotor is to be increased above a limit value, in order that rotating effect should have proper torque, i.e. a properly dimensioned power trasmitting arm.

Bending of the flange delimits increasing the diameter of the rotor, this can be counterbalanced by increasing thickness, however this results in increase in weight and friction. Diameter of the apparatus 20 will be restricted by the fact, too, that it could be used as comfortable means. According to our experiments, when using a PVC rotor, practically a diameter of the rotor of 7 to 8 cm proved to be the best. Weight of the rotor may amount to 0,1-2 g the most, preferably 0,3 to 0,8 g.

In figure 2 a preferable embodiment is shown, being suitable for decreasing friction and for increasing sensitivity. In the centre of the rotor 3 the hub 4 is inserted which is elevated above the plane of the rotor 3 and comes down in a bell-like manner onto the shaft 2, accordingly stable suspension is assured for the rotor 3. Preferably the hub 4 is lined with a hard material, e.g. a carrier 5 made of agate and fits with its calotte-shaped surface to the peak of the shaft 2.

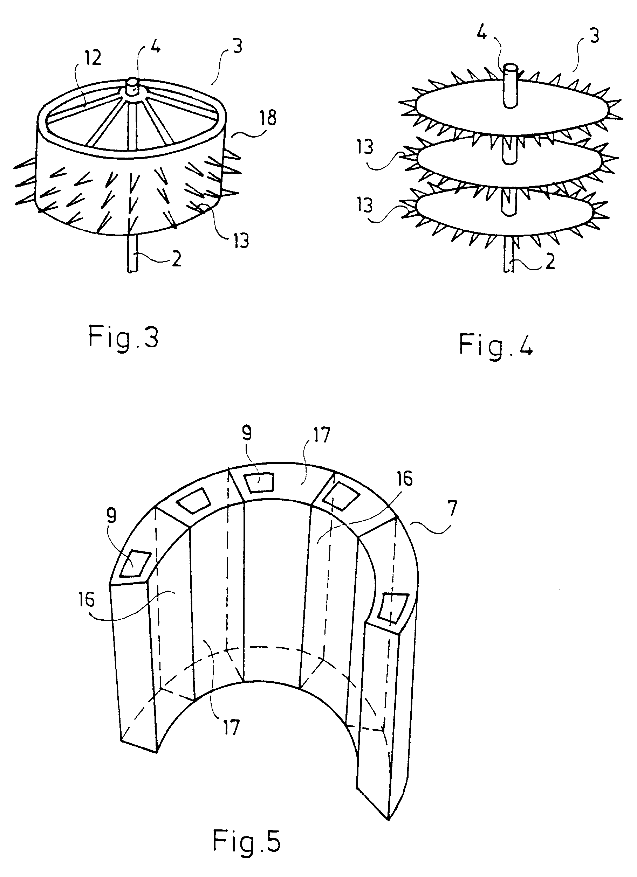

Figure 3 illustrates another possible embodiment, with which a wide cylinder-jacket is arranged circumferentially on the edge of the rotor 3, forming the mantle 18 hanging down from the spoked wheel. As it becomes obvious from figure 3, spokes 12 are not unconditionally planar, in this case they form expediently a conical mantle having the hub in its peak.

Obtainable number of revolutions can be further increased, if on the circumference of the edge of the rotor 3 one or more arbors 13 (in the latter case arranged in rows next to each other) are formed, directed to the stator 1. Size of the arbors 13 amounts expediently to 3 to 4 mm, while the angle of vertex (cuspidity) must not exceed 15°.

According to the example detailed here, the rotor is formed with a 7,5 cm diameter, the size of the mantle 18 equals preferably to 2-3 cm, lying parallel with the diameter. Simultaneous effect of the mantle 18 and the arbors 13 increase obtainable number of revolutions by about 25-30%, that means that the apparatus is able to respond. By using 32 pcs of arbors within a circle, an improvement of 25% can be already achieved, while with 64 arbors 30% improvement becomes possible. It is not worth while to form arbor-rows more, than five, as no surplus-effect can be achieved, optimally three rows seem to be the best.

With another preferred embodiment the rotor 3 is composed of a plurality of discs or wheels with spokes 12, which are arranged axially, one below the other on the properly extended hub 4. Similarly to the previously described embodiment, edge of said discs is also provided with the arbors 13. With a rotor 3 consisting of three discs and considered as an optimal solution, number of revolutions is by 10% higher, than with one single disc. It seems to be expedient to use discs with identical diameters to be arranged parallel with each other in equal mutual distances. According to our experiences having been gained in course of experiments, change in the latter factors does not exert any meritory effect on the result.

Application of the rotor with a plurality of discs does not appear merely in increased number of revolution, rather in the improved stability of the apparatus. Possible disturbing effects, as draught, can be far better eliminated by using the aforementioned embodiment. Number of revolutions is rendered stable, function of the apparatus 20 becomes more stabilized, too.

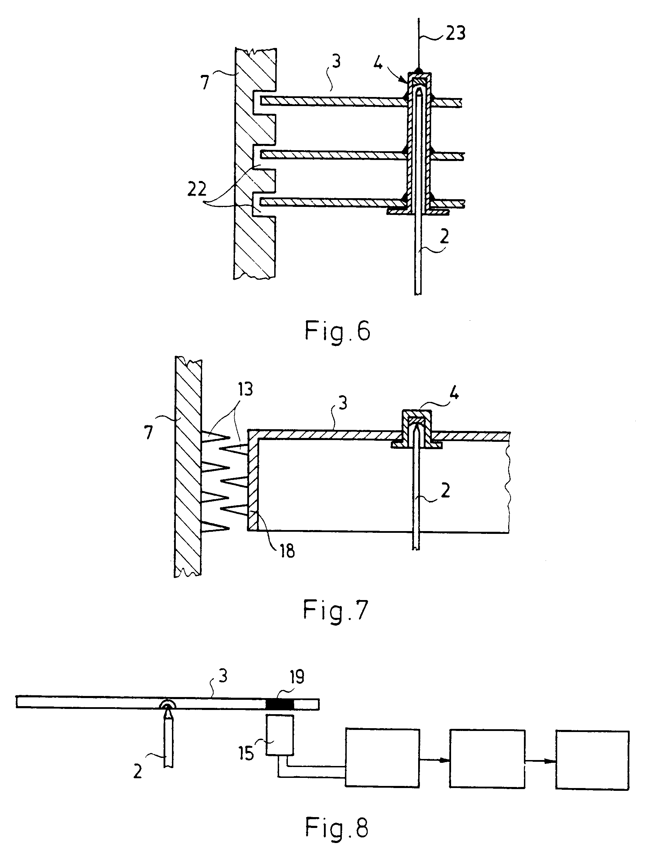

Figures 6 and 7 propose further solutions for increasing number of revolutions and improving sensitivity. Essential feature of both solutions lies in that the stator 1, more accurately the block 7 thereof will show some advantageous formation on the inner wall, in the height of the rotor 3. In figure 6 a multi-disc rotor 3 as illustrated already in figure 4 is to be seen, having a block 7, on the inner wall of which recesses 22 were formed, the edge of the rotor 3 extends in said recesses. Even with a single-disc rotor 3 it is worth while to form the recesses 22. In this case the recess 22 receiving said single-disc rotor is enclosed on both sides by one or two further recesses each.

In this case the hub 4 is not bearing up against the shaft 2, but it is suspended on the torsional filament 23. Practically this solution is free of friction and it is well suitable for the display of trifle moments. Of course, the shaft 2 can be replaced by another filament. Thereby a strained structure can be constructed.

Referring now to figure 7, rows of arbors of the rotor 3, provided with the mantle 18 - as already seen in figure 3 - are fitting in between the similar arbors 13 on the inner wall of the block facing the rotor, in course of the rotary motion rows of arbors are passing between one another. Aforementioned favourable effect of the arbors 13 asserts itsel ffully by the virtue of the advantageous formation of the block 7.

As an expedient development of the stator 1 represents a subdivision of the cavity 17 by means of the lamellae 16 (figure 5). In this case separate filling means 9 are to be provided for for every single part within the separated cavity 17. Preferably, number of lamellae 16 makes out four, it is not worth while to divide the cavity in parts exceeding five.

Supposing a 7 to 8 cm diameter of the rotor, cylindrical height of the stator amounts expediently to 10 cm. By doubling the height of the stator, 100 percentual increase in number of revolutions can be achieved, at the same time dimensions and manipulation of the apparatus 20 will be influenced negatively.

Sign 19 on the rotor, which might be a coloured strip or a spot, facilitates reading of r.p.m. The equipment can be easily completed with any type of automatic revolution counter known in itself. A possible arrangement is shown in figure 8. If the rotor 3 is made of some transparent material, the sign 19 on the rotor 3 is a covered opaque section on the edge of the rotor 3. In contrast to that if the rotor 3 is made of some opaque material, the sign 19 appears as a perforation. The photodiode having been arranged below the rotor 3 detects the change in light resulting from the travel of the sign 19, said change will be displayed by the connected pulse-counter, processor and dsiplay, indicating and measuring number of revolutions. (Counter of revolution does not require any light source, as the light on the place of testing is sufficient for activating the photodiode). If the edge of the rotor 3 carries more, than one sign 19, automatic measurement and display can be well used for measuring fractional revolutions.

By using the modifications, as described in connection with examples, maximal r.p.m. can be achieved with the apparatus. Increasing the value of maximal revolutions per minute simultaneously widens the circle of persons, the proximity of which puts the apparatus into motion, at all. Most simple embodiment of the apparatus 20 is able to yield a value of 6 to 8 r.p.m. in such a manner at the majority of the persons tested not even one single revolution can be achieved. By introducing different improved versions of the stator 1 and the rotor 3, respectively, peak of revolutions can be increased quite up to thirty and the majority of the persons tested - supposedly being in a good health - may release at least one revolution.

Application of the method according to the invention will be detailed by means of some examples.

### Example 1

Examination is performed by means of the apparatus, as seen in figure 1, containing a single-disc rotor 3 with a diameter of 7,5 cm and a weight (mass) of 0,7 g. Neither filler, nor heightening of the cylinder were used with the stator 1.

Apparatus 20 was set up at the place of testing and we observed it for 5 to 10 minutes. Rotor 3 was in stillstand.

After having approached the outside of the block 7 with a body made of lead and having a considerable mass and keeping it next to the apparatus for 4 to 5 minutes, no change could be observed. Similarly, no change could be observed by using an iron body (a dumb-bell) or a heated iron ball.

We approached the block 7 of the stator and the window 8 from all sides with a rubber-glove filled with hot water, neither rotation, nor motion could be observed.

No result could be achieved, when specimens were brought into contact with the outer part of the block 7, or - in case of cold specimens - with the window 8 made of plexiglass.

In the proximity of the apparatus 20 a strong electromagnetic field was induced by means of a solenoide, however, rotation of the rotor 3 could not be achieved by any means.

After having introduced an air-current, shot vigorously into the inside of the apparatus 20 through the airgap 6, the rotor 3 tilted in some direction or deflected, possibly it made one turn, but after having ceased blowing, the rotor returned into stillstand.

### Example 2

By using the embodiment of the apparatus 20 as specified in example 1, we tested about 1600 persons for collecting statistical data. The group of persons tested included people of different age, profession and educational level in an equal proportion. Prior to testing, state of health and fitness were controlled by a medical specialist, partly by ocular inspection, on basis of thermometer records, in several cases by measuring blood-pressure.

On basis of subjective opinion, the persons tested reported on their diseases and momentary condition of health.

Prior to testing we set up the apparatus on a place being free of draught and vibration, after every 10 to 15 tests we controlled the arrangement for 5 minutes each.

Tests were performed so, as the person to be tested was sitting opposite to the window 8 of the stator of the apparatus, reaching out his/her arm to the other side of the apparatus and bending his/her hand at the writs, simultaneously putting the bent fingers around the block 7. As for the results, according to experiences it was quite neutral, whether he/she touched the apparatus or held the hand next to it.

Based on 1600 tests the following experimental scale could be observed:
- O turns:: in case of serious illness or considerable fatigue, deconcentration (scattered attention)
- 0 to 2 turns:: sleepiness in the morning, lack of sleep, some disease in the initial stage
- 2 to 4 turns:: fatigue, effect of fronts, inferior ability of concentration
- 4 to 6 turns:: average condition of health
- 6 to 10 turns:: peak condition in dependence of individual characteristics
- 10 to 16 turns:: after a long rest, the state of full concentration
- 16 to 20 turns:: exceptionally good conditions, individual characteristics and ability of concentration surpasses the average
- above 20 turns:: extraordinary abilities, mostly infrequent physical and mental features.

Highest value having been measured in course of examination was 28, tests were repeated with the person yielding this value, the result was not erroneous at all, in a fully relaxed state the aforementioned value could be reproduced without exception.

### Example 3

By using the version of the apparatus 20 according to Example 1 and applying the method in accordance with Example 2, we performed tests in two classes of a secondary school, totally with 45 persons, boys and girsl of the age of 15 to 17 years. Tests were performed for three weeks, three times a week, first in the morning, when arriving at school, next after lunch. Children having a flu or headache or with similar complaints were left out. Pupils with long-lasting illnesses were not subjected to the tests, although they attended the school.

Results obtained were as follows:
0 turn with two persons
1 to 3 turns with five persons
4 to 5 turns with 25 persons
6 to 7 turns with 11 persons
11 turns with one person
16 turns with one person.

Results were evaluated at the end of the third week, commonly with teachers and pupils. The girl with sixteen turns was a mediocre pupil, but taking regularly part in gymnastic competition of first class, eleven turns were achieved by a boy being active in computer programming on a semiprofessional level. Out of the 45 persons among the 13 children yielding turns above five, proportion of good scholars was not higher, than among the 32 persons with turns under five, among the 13 persons all the individuals could be found, which gave a better result, than the mediocre level, achieving it with inconsiderable diligence and work. Although in the group with turns less, than six, pupils with very good marks could be found; those children, who exerted a significant effect on their environment and their classmates, that means who were considered as personalities both by their classmates and teachers, reached at least six turns.

### Example 4

Tests were performed in a home for pensioners under steady medical supervision; 37 persons were subjected to tests, in the age between 58 and 84 years, for five months and once a week, using the apparatus and method, as described in previous examples.

In course of initial measurements we obtained 1 to 3 turns with six persons, with the rest none. Conversations with older people and our interest shown towards them resulted in a positive effect, with the majority, i.e. with twenty-eight persons 1 to 3 turns could be measured on occasion of the second-third test. Simultaneously, on basis of medical examinations less and greater complaints and illnesses were treated with medicaments, i.e. earlier treatments were substituted by new medicaments or therapeutic methods. With sixteen aforementioned persions measurable values could be increased from 1 to 2 turns to 3 to 4. With further twelve persons no kind of somatic (physical) motifs could be observed, merely mental symptoms. These persons received medicaments with "placebo" character, thereafter turns increased from 1 to 2 to 3-4 turns. Further six persons were similarly treated with placebo, however measuring results did not improve, the more, with three persons they fell back permanently to zero. In course of more thorough examinations with six persons earlier not recognized somatic diseases could be detected. With progress of necessary treatments our measuring results showed an improvement of 2-3 turns being characteristic for the majority after three-five measurements. With three persons a permanent zero-level of turns could be observed, later we were informed that they were of failing health due to serious deterioration by their age.

### Example 5

We performed a series of tests in a psychologic club, using the earlier specified apparatus and methods. In this club mainly meditation and relaxation were taught and practiced.

For experimental purposes with newly joining 33 persons application of the apparatus according to the invention was introduced. A group consisting of eighteen persons - as control group - met the measuring method only at the beginning of the training, while another group of fifteen persons used the process according to the invention for self-control in course of meditation and relaxing practice.

First measurements showed that 33 persons produced turns between 2 and 6, distribution of individual characteristics showed essentially corresponding statistic distribution with both the experimental and the control group.

Training program lasts averagely six weeks each. According to the opinion of the club leaders members of the experimental group aquired the abilities obtainable on the fifth or sixth week already at the end of the third week. At the same time, the third week our tests gave the following results:

| | |
|---|---|
| 1 person | 21 turns |
| 3 persons | 16 to 20 turns |
| 7 persons | 10 to 16 turns |
| 4 persons | 7 to 9 turns. |

Members of the control group finished the training in the fifth-sixth week and reached the desired ability of meditation. Relating data are as follows:

| | |
|---|---|
| 2 persons | 19 turns |
| 9 persons | 10 to 17 turns |
| 7 persons | 6 to 9 turns. |

In course of common evaluation we arrived at the conclusion, in so far as the members of the experimental group were able to control the development of their capabilities, in such a manner their practices became more conscious and more concentrated. As a consequence improvement was quicker and more succesful.

Advantages of the invention can be summarized as follows.

The apparatus is simple and cheap, a properly sensitive version can be realized even with a simple embodiment.

Operation does not require external energy sources, it is portable and can be set up without any difficulty. Putting into operation takes place quickly.

The apparatus yields well definable numerical values characterizing health and fitness.

In case of regularly performed measurings with one and the same person, success and efficiency of some cure or training can be controlled.

At the same time striking changes appearing in results of regularly performed measurings may call the attention to some physical or mental deterioration or impending disease.

In case of measurings having been performed in a circle of several persons, results may call the attention to exhausted and sick persons, who require more attention to extraordinarily talented persons with increased ability for concentration, having favourable physical and mental natural endowments, who may be excessively successful on certain fields (result may be used as a factor in talent spotting).

## Claims

1. Apparatus for demonstrating and measuring physical effects being in connection with the general state of health and fitness of human beings, **characterized** by comprising a rotor (3) arranged rotatably on a frame (11), of the mass of 0,1 to 2,0 g, more accurately 0,3 to 0,8 g, with at least one marking (19) on the edge cooperating with means for reading number of turns, wherein relative magnetic permeability is less than 50, and in which furtheron optionally the rotor (3) and the frame (11) are connected to each other by a spring means.

2. Apparatus as claimed in claim 1, **characterized** in that around the rotor (3) there is a stator (1) of the shape of a cylindrical jacket comprising a block (7) made of metal and a window (8) made of a non-metallic material and provided with an airgap (6), wherein said block (7) forms a circular arc of the stator (1) at an angle of 160° to 200°.

3. Apparatus as claimed in claim 1 or 2, **characterized** in that relative dielectric constant of the rotor (3) is higher than 2,5.

4. Apparatus as claimed in any of the claims 1 to 3, **characterized** in that the rotor (3) is made of paper.

5. Apparatus as claimed in any of the claims 1 to 3, **characterized** in that the rotor (3) is made of some synthetic material.

6. Apparatus as claimed in any of the claims 1 to 5, **characterized** in that magnetic permeability of the rotor is less than 1.

7. Apparatus as claimed in any of the claims 1 to 6, **characterized** in that the rotor is shaped as a lightened disc.

8. Apparatus as claimed in any of the claims 1 to 7, **characterized** in that the frame (11) comprises of a stand (21) and a shaft (2).

9. Apparatus as claimed in claim 7, **characterized** in that on the circumference of the edge of the rotor (3) a mantle (18) of the shape of a cylinder-jacket is arranged and the generatrices of which are parallel with the shaft (2).

10. Apparatus as claimed in any of the claims 1 to 6, **characterized** in that in the centre of the rotor (3) there is a hub (4) being lined with a carrier (5) made of some hard material, e.g. agate and has the shape of a calotte, while the rotor is bearing up against the shaft (2) with the intervention of the carrier (5) and the hub (4).

11. Apparatus according to any of the claims 2 to 10, **characterized** in that specific weight of the block (7) amounts to at least 7.

12. Apparatus as claimed in any of the claims 2 to 11, **characterized** in that relative magnetic permeability of the block (7) is at least 2.10⁴.

13. Apparatus as claimed in any of the claims 2 to 12, **characterized** in that the double-walled block (7) is provided with filling means (9) and the cavity of the block is filled with some filler, so e.g. water, oil or sugar.

14. Apparatus as claimed in claim 13, **characterized** in that the cavity is subdivided into a plurality of independent parts by means of lamellae (16).

15. Apparatus as claimed in any of the claims 2 to 14, **characterized** in that circumferentially on the edge of the rotor (3) one or more arbors (13) are arranged, in the latter case in one or more rows below each other directed to the stator (1) and on the inner wall of the block (7), in the height of the rotor (3) facing the rotor (3), wherein the rows of arbors of the rotor (3) are arranged between the arbor-rows on the block (7) and vice versa.

16. Apparatus as claimed in any of the claims 2 to 14, **characterized** in that on the inner wall of the block (7) circumferentially, in the height of the rotor (3) recesses (22) are formed and edge(s) of the rotor (3) penetrate into said recess(es) (22).

17. Apparatus as claimed in any of the claims 1 to 16, **characterized** in that it is provided with a rotation-detector (display) known in itself, and so, that on the rotor (3) made of some transparent material one or more light-shutting-off spots or strip-like markings (19) are to be found, while on the opaque rotor (3) one or more perforations (19) are formed, below the path of rotation of the markings (19) there is a light-sensitive photodiode, connected to a pulse-counter, a processor and a display unit.

18. Method for measuring and demonstrating physical effects appearing on body-parts being in connection with general conditions of health and fitness of human beings, **characterized** by providing the apparatus according to claims 1 to 17 comprising a rotor (3) arranged rotatably on a frame (11), of the mass of 0,1 to 2,0 g, more accurately 0,3 to 0,8 g, with at least one marking (19) on the edge cooperating with means for reading number of turns, wherein relative magnetic permeability is less than 50, and in which furtheron optionally the rotor (3) and the frame (11) are connected to each other by a spring means, placing the apparatus on a draught- and vibration free place, placing a member of the body of the person to be tested, e.g. the hand next to the apparatus (20) in a distance of 8 cm or less for a period of at least 4-5 minutes, thereafter reading the stabilized number of revolutions of the rotor (3) or deflection from its stillstand.

## Patentansprüche

1. Vorrichtung zur Demonstration und zum Messen physischer Effekte, die mit dem allgemeinen Gesundheitszustand und der Fitness menschlicher Lebewesen zusammenhängen, gekennzeichnet durch das Aufweisen eines drehbar auf einem Rahmen (11) angeordneten Rotors (3) mit einem Gewicht von 0,1 bis 2,0 g, genauer 0,3 bis 0,8 g, der wenigstens eine Markierung (19) an dem Rand aufweist, die mit Mitteln zum Ablesen der Anzahl von Umdrehungen zusammenarbeitet, wobei die relative magnetische Permeabilität weniger als 50 beträgt, und in welcher weiter der Rotor (3) und der Rahmen (11) bedarfsweise mittels eines Federmittels miteinander verbunden sind.

2. Vorrichtung nach Anspruch 1, dadurch gekennzeichnet, daß rings des Motors (3) ein Stator in Form einer zylindrischen Umhüllung angeordnet ist, die einen aus Metall hergestellten Block (7) und ein aus einem nicht-metallischen Material hergestelltes und mit einem Luftspalt (6) versehenes Fenster (8) aufweist, wobei der Block (7) einen kreisförmigen Bogen des Stators mit einem Winkel von 160° bis 200° ausbildet.

3. Vorrichtung nach Anspruch 1 oder 2, dadurch gekennzeichnet, daß die relative dielelektrische Konstante des Rotors größer als 2,5 ist.

4. Vorrichtung nach einem der Ansprüche 1 bis 3, dadurch gekennzeichnet, daß der Rotor (3) aus Papier hergestellt ist.

5. Vorrichtung nach einem der Ansprüche 1 bis 3, dadurch gekennzeichnet, daß der Rotor (3) aus synthetischem Material hergestellt ist.

6. Vorrichtung nach einem der Ansprüche 1 bis 5, dadurch gekennzeichnet, daß die magnetische Permeabilität des Rotors kleiner als 1 ist.

7. Vorrichtung nach einem der Ansprüche 1 bis 6, dadurch gekennzeichnet, daß der Rotor als leichter gemachte Scheibe ausgebildet ist.

8. Vorrichtung nach einem der Ansprüche 1 bis 7, dadurch gekennzeichnet, daß der Rahmen (11) einen Fuß (21) und einen Schaft (2) aufweist.

9. Vorrichtung nach Anspruch 7, dadurch gekennzeichnet, daß an dem Umfangsrand des Rotors (3) ein Kranz in der Form einer Zylinderummantelung angeordent ist, und deren Erzeugende parallel zu dem Schaft sind.

10. Vorrichtung nach einem der Ansprüche 1 bis 6, dadurch gekennzeichnet, daß im Zentrum des Rotors (3) eine Nabe (4) mit einem Träger (5) aus irgendeinem harten Material, z.B. Achat, ist und die Form einer Kalotte hat, während der Rotor über den Träger (5) und die Nabe (4) auf dem Schaft (2) gelagert ist.

11. Vorrichtung nach einem der Ansprüche 2 bis 10, dadurch gekennzeichnet, daß das spezifische Gewicht des Blocks (7) wenigstens 7 beträgt.

12. Vorrichtung nach einem der Ansprüche 2 bis 11, dadurch gekennzeichnet, daß die relative magnetische Permeabilität des Blocks wenigstens 2.10⁴ ist.

13. Vorrichtung nach einem der Ansprüche 2 bis 12, dadurch gekennzeichnet, daß der doppelwandige Block (7) Füllmittel (9) aufweist und der Hohlraum des Blocks mit einem Füllmaterial wie z.B. Wasser, Öl oder Zucker gefüllt ist.

14. Vorrichtung nach Anspruch 13, dadurch gekennzeichnet, daß der Hohlraum mittels Lamellen (16) in mehrere unabhängige Abschnitte unterteilt ist.

15. Vorrichtung nach einem der Ansprüche 2 bis 14, dadurch gekennzeichnet, daß rings des Umfangsrandes des Rotors (3) eine oder mehrere Dorne (13) angeordnet sind, im letzten Fall in einer oder mehreren Reihen untereinander auf den Stator gerichtet, und auf der inneren Wandung des Blocks (7), dem Rotor (3) auf Höhe des Rotors (3) gegenüberliegend, wobei die Reihen von Dornen des Rotors (3) zwischen den Dorn-Reihen an dem Block (7) und umgekehrt angeordnet sind.

16. Vorrichtung nach einem der Ansprüche 2 bis 14, dadurch gekennzeichnet, daß in der inneren Wandung des Blocks (7) auf Höhe des Rotors Umfangsaussparungen (22) ausgebildet sind, und der Rand/Ränder des Rotors (3) in die Aussparung/Aussparungen (22) hineinragt.

17. Vorrichtung nach einem der Ansprüche 1 bis 16, dadurch gekennzeichnet, daß diese einen Umdrehungs-Detektor (Anzeige), der an sich bekannt ist, derart aufweist, daß auf dem aus einem transparenten Material hergestellten Rotor (3) eine oder mehrere Licht-unterbrechende punkt- oder streifenförmige Markierungen (19) vorgesehen sind, wohingegen an dem opaken Rotor (3) ein oder mehrere Lochungen (19) ausgebildet sind, wobei unter der Umlaufbahn der Markierungen (19) eine lichtempfindliche Photodiode angeordnet ist, die mit einem Impulszähler, einem Prozessor und einer Anzeigeeinheit verbunden ist.

18. Verfahren zur Demonstration und zum Messen physischer Effekte, die mit dem allgemeinen Gesundheitszustand und der Fitness menschlicher Lebewesen zusammenhängen, gekennzeichnet durch das Vorsehen der Vorrichtung nach Ansprüchen 1 bis 17, die einen drehbar auf einem Rahmen (11) angeordneten Rotor (3) mit einem Gewicht von 0,1 bis 2,0 g, genauer 0,3 bis 0,8 g aufweist, der wenigstens eine Markierung (19) an dem Rand aufweist, die mit Mitteln zum Ablesen der Anzahl von Umdrehungen zusammenarbeitet, wobei die relative magnetische Permeabilität weniger als 50 beträgt, und in welcher weiter der Rotor (3) und der Rahmen (11) bedarfsweise mittels eines Federmittels miteinander verbunden sind, wobei die Vorrichtung an einer luftzug- und erschütterungsfreien Stelle plaziert wird, wobei eine Körperteil, z.B. die Hand der Testperson nahe der Vorrichtung in einem Abstand von 8 cm oder weniger für eine Zeitspanne von 4-5 Minuten plaziert wird, wobei danach die stabilisierte Anzahl der Umdrehungen des Rotors (3) oder Abweichungen von dessen Stillstand abgelesen werden.

## Revendications

1. Appareil pour démontrer et mesurer des effets physiques en relation avec l'état général de santé et d'état physique d'êtres humains, caractérisé en ce qu'il comprend un rotor (3) monté à rotation sur un cadre (11), d'une masse de 0,1 à 2,0 g, plus exactement de 0,3 à 0,8 g, avec au moins une marque (19) sur le bord coopérant avec un moyen de lecture de nombre de tours, dans lequel la perméabilité magnétique relative est inférieure à 50, et dans lequel de plus le rotor (3) et le cadre (11) sont facultativement reliés l'un à l'autre par un moyen de ressort.

2. Appareil selon la revendication 1, caractérisé en ce qu'autour du rotor (3) il y a un stator (1) ayant la forme d'une enveloppe cylindrique comprenant un bloc (7) fait de métal et une fenêtre (8) faite d'un matériau non métallique et pourvue d'un espacement d'air (6), dans lequel ledit bloc (7) forme un arc circulaire du stator (1) suivant un angle de 160° à 200°.

3. Appareil selon la revendication 1 ou 2, caractérisé en ce que la constante diélectrique relative du rotor (3) est supérieure à 2,5.

4. Appareil selon l'une quelconque des revendications 1 à 3, caractérisé en ce que le rotor (3) est fait en papier.

5. Appareil selon l'une quelconque des revendications 1 à 3, caractérisé en ce que le rotor (3) est fait de matériau synthétique.

6. Appareil selon l'une quelconque des revendications 1 à 5, caractérisé en ce que la perméabilité magnétique du rotor est inférieure à 1.

7. Appareil selon l'une quelconque des revendications 1 à 6, caractérisé en ce que le rotor a la forme d'un disque allégé.

8. Appareil selon l'une quelconque des revendications 1 à 7, caractérisé en ce que le cadre (11) comprend un support (21) et un arbre (2).

9. Appareil selon la revendication 7, caractérisé en ce que sur la circonférence du bord du rotor (3) est disposé un manchon (18) ayant la forme d'une enveloppe de cylindre et dont les génératrices sont parallèles à l'arbre (2).

10. Appareil selon l'une quelconque des revendications 1 à 6, caractérisé en ce qu'au centre du rotor (3) il y a un moyeu (4) garni d'un support (5) fait d'un matériau dur, par exemple de l'agate et ayant la forme d'une calotte, tandis que le rotor porte sur l'arbre (2) par l'intermédiaire du support (5) et du moyeu (4).

11. Appareil selon l'une quelconque des revendications 2 à 10, caractérisé en ce que le poids spécifique du bloc (7) se monte à au moins 7.

12. Appareil selon l'une quelconque des revendications 2 à 11, caractérisé en ce que la perméabilité magnétique relative du bloc (7) est d'au moins 2⁻10⁴.

13. Appareil selon l'une quelconque des revendications 2 à 12, caractérisé en ce que le bloc à double paroi (7) est pourvu de moyens de remplissage (9) et la cavité du bloc est remplie d'un certain produit de remplissage, par exemple de l'eau, de l'huile ou du sucre.

14. Appareil selon la revendication 13, caractérisé en ce que la cavité est subdivisée en une pluralité de parties indépendantes au moyen de lamelles (16).

15. Appareil selon l'une quelconque des revendications 2 à 14, caractérisé en ce qu'une ou plusieurs dents (13) sont disposées périphériquement sur le bord du rotor (3), dans le dernier cas suivant une ou plusieurs lignes les unes en dessous des autres dirigées vers le stator (1) et sur la paroi intérieure du bloc (7), sur la hauteur du rotor (3) faisant face au rotor (3), les lignes de dents du rotor (3) étant disposées entre les lignes de dents sur le bloc (7) et vice versa.

16. Appareil selon l'une quelconque des revendications 2 à 14, caractérisé en ce que des évidements (22) sont formés périphériquement dans la paroi intérieure du bloc (7), dans la hauteur du rotor (3), et un (des) bord(s) du rotor (3) pénètre(nt) dans le(s)dit(s) évidement(s) (22).

17. Appareil selon l'une quelconque des revendications 1 à 16, caractérisé en ce qu'il est pourvu d'un détecteur de rotation (affichage) connu en lui-même, et de telle façon que sur le rotor (3) fait d'un matériau transparent se trouvent une ou plusieurs marques de coupure de lumière en forme de points ou de bandes, tandis que sur le rotor opaque (3) sont formées une ou plusieurs perforations (19), sous le trajet de rotation des marques (19) il y a une photodiode sensible à la lumière, reliée à un compteur d'impulsions, un processeur et une unité d'affichage.

18. Procédé pour mesurer et démontrer des effets physiques apparaissant sur des parties du corps en relation avec des conditions générales de santé et d'état physique d'êtres humains, caractérisé en ce qu'on utilise l'appareil selon les revendications 1 à 17 comprenant un rotor (3) monté à rotation sur un cadre (11), d'une masse de 0,1 à 2,0 g, plus exactement de 0,3 à 0,8 g, avec au moins une marque (19) sur le bord coopérant avec un moyen de lecture de nombre de tours, dans lequel la perméabilité magnétique relative est inférieure à 50, et dans lequel de plus le rotor (3) et le cadre (11) sont facultativement reliés l'un à l'autre par un moyen de ressort, on place l'appareil en un emplacement exempt de courants d'air et de vibrations, on place un élément du corps de la personne à tester, par exemple la main proche de l'appareil (20) à une distance de 8 cm ou moins pendant une période d'au moins 4-5 minutes, on lit ensuite le nombre stabilisé de tours du rotor (3) ou la déflexion à partir de son état de repos.
